# EUROPEAN PATENT APPLICATION

(11) **EP 4 803 509 A1**
(43) Date of publication of application: **09.09.2026**
(21) Application number: 24885815.1
(22) Date of filing: 31.10.2024
(51) Int. Cl.: C07D 265/30, A61K 8/49, A61Q 17/04, A61Q 19/00, C07D 265/36, C07D 413/06, C07D 417/06

(54) **NOVEL VINAMIDINIUM SALT AND UV-A ABSORBENT CONTAINING SAID NOVEL VINAMIDINIUM SALT**

(30) Priority: 02.11.2023 JP 2023188088
(71) Applicant: Kyushu University, National University Corporation, Nishi-ku Fukuoka-shi Fukuoka 819-0395 (JP)
(72) Inventor: SHINDO, Mitsuru, Fukuoka-shi, Fukuoka 819-0395 (JP); IWATA, Takayuki, Fukuoka-shi, Fukuoka 819-0395 (JP)
(74) Representative: J A Kemp LLP
(86) International application number: PCT/JP2024/038851
(87) International publication number: WO 2025/095048

(57) **Abstract**

A novel vinamidinium salt shown by the following formula (1). 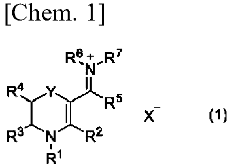 (in formula (1), R¹, R², R⁵, R⁶ and R⁷ each independently represents a hydrogen atom, an alkyl group with 1 to 10 carbon atoms optionally having a substituent, etc.; R³ and R⁴ each independently represents a hydrogen atom, an alkyl group with 1 to 10 carbon atoms optionally having a substituent, etc.; Y is O, S, NR⁸, CR⁸R⁹; R⁸ and R⁹ each independently represents a hydrogen atom, an alkyl group with 1 to 10 carbon atoms optionally having a substituent, etc.; R¹ to R⁹ can form a cyclic structure by binding to each other; and X⁻ represents an anion).

## Description

### TECHNICAL FIELD

The present invention relates to a novel vinamidinium salt, and an UV-A absorbent comprising such novel vinamidinium salt.

### BACKGROUND ART

Ultraviolet ray comprised in sunray is classified into UV-A (320-400 nm), UV-B (280-320 nm), UV-C (200-280 nm) according to wavelength. Among these, UV-A and UV-B have effects on living organisms, while UV-B has stronger energy than UV-A, and easily cause sunburn. Therefore, the main ingredients of conventional sunscreen agents available in the market were mostly absorbent and reflecting agent of UV-B.

However, since UV-A reaches dermis without being absorbed by the ozone layer, it has been revealed to be the cause of wrinkling or sagging, and it is also important to protect skin from UV-A.

As conventional UV-A absorbent, merely several types of benzene-based compounds that were concerned to cause skin irritation as side-effect were known. Further, non-benzene type mycosporine-like amino acids are reported, but were not sufficient since their absorbing ability is weak, the maximal absorption wavelength is short wavelength, and acidic condition is essential (see Non-patent Literature 1).

On the other hand, methylobamine, a microorganism-derived natural organic compound, is a non-benzene type hydrophilic small molecule, and it is reported to have an excellent UV-A absorbency (see Patent Literature 1). However, as it is a natural trace ingredient, difficult to obtain, their properties detail were not elucidated and supply by chemical synthesis was difficult.

### CITATION LIST

### Non-Patent Literatures

Non-Patent Literature 1: Vanessa Geraldes, Ernani Pinto, Mycosporine-Like Amino Acids (MAAs): Biology, Chemistry and Identification Features.2021

### Patent Literatures

Patent Literature 1: Japanese Patent No. 5751517

### SUMMARY OF INVENTION

### TECHNICAL PROBLEM

The object of the present invention is to provide a novel vinamidinium salt useful as UV-A absorbent.

### SOLUTION TO PROBLEM

The present inventors found out that vinamidinium salt having a particular structure has an excellent UV-A absorbing property, and the present invention has been thus completed.

Specifically, the present invention is as follows.
[1] A vinamidinium salt shown by the following formula (1): 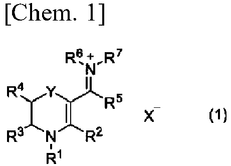
   (in formula (1)
   R¹, R², R⁵, R⁶ and R⁷ each independently represents a hydrogen atom, an alkyl group with 1 to 10 carbon atoms optionally having a substituent, an alkenyl group with 1 to 10 carbon atoms optionally having a substituent, an alkynyl group with 1 to 10 carbon atoms optionally having a substituent, an aryl group with 6 to 14 carbon atoms optionally having a substituent, an aralkyl group with 7 to 20 carbon atoms optionally having a substituent, or a hydroxy group;
   R³ and R⁴ each independently represents a hydrogen atom, an alkyl group with 1 to 10 carbon atoms optionally having a substituent, an alkenyl group with 1 to 10 carbon atoms optionally having a substituent, an alkynyl group with 1 to 10 carbon atoms optionally having a substituent, an alkoxy group with 1 to 10 carbon atoms optionally having a substituent, a hydroxy group or a halogen atom;
   Y is O, S, NR⁸, CR⁸R⁹;
   R⁸ and R⁹ each independently represents a hydrogen atom, an alkyl group with 1 to 10 carbon atoms optionally having a substituent, an alkenyl group with 1 to 10 carbon atoms optionally having a substituent, an alkynyl group with 1 to 10 carbon atoms optionally having a substituent, an alkoxy group with 1 to 10 carbon atoms optionally having a substituent, an aryl group with 6 to 14 carbon atoms optionally having a substituent, an aralkyl group with 7 to 20 carbon atoms optionally having a substituent, or a hydroxy group;
   R¹ to R⁹ can form a cyclic structure by binding to each other; and
   X⁻ represents an anion).
[2] The vinamidinium salt according to the above [1], wherein R⁶ and R⁷ constitute a nitrogen-containing cyclic group together with a nitrogen atom to which they are bonded.
[3] The vinamidinium salt according to the above [2], wherein the nitrogen-containing cyclic group is at least one group selected from an azetidine group, a pyrrolidine group, a piperidine group, an azepane group, a morpholine group, a thiomorpholine group, a thiomorpholine dioxide group, and a piperazine group.
[4] The vinamidinium salt according to any one of the above [1] to [3], wherein the vinamidinium salt is shown by the following formula (1A): 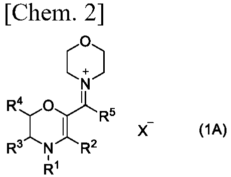
[5] The vinamidinium salt according to the above [4], wherein in formula (1A), R¹ represents an alkyl group with 1 to 4 carbon atoms, R² represents a methyl group, R³ to R⁵ represent a hydrogen atom, and X⁻ represents PF₆⁻.
[6] A vinamidinium salt shown by the following formula (2). 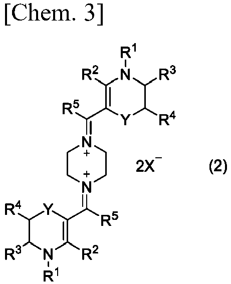
   (in formula (2),
   R¹, R² and R⁵ each independently represents a hydrogen atom, an alkyl group with 1 to 10 carbon atoms optionally having a substituent, an alkenyl group with 1 to 10 carbon atoms optionally having a substituent, an alkynyl group with 1 to 10 carbon atoms optionally having a substituent, an aryl group with 6 to 14 carbon atoms optionally having a substituent, an aralkyl group with 7 to 20 carbon atoms optionally having a substituent, or a hydroxy group;
   R³ and R⁴ each independently represents a hydrogen atom, an alkyl group with 1 to 10 carbon atoms optionally having a substituent, an alkenyl group with 1 to 10 carbon atoms optionally having a substituent, an alkynyl group with 1 to 10 carbon atoms optionally having a substituent, an alkoxy group with 1 to 10 carbon atoms optionally having a substituent, a hydroxy group or a halogen atom;
   Y is O, S, NR⁸, CR⁸R⁹;
   R⁸ and R⁹ each independently represents a hydrogen atom, an alkyl group with 1 to 10 carbon atoms optionally having a substituent, an alkenyl group with 1 to 10 carbon atoms optionally having a substituent, an alkynyl group with 1 to 10 carbon atoms optionally having a substituent, an alkoxy group with 1 to 10 carbon atoms optionally having a substituent, an aryl group with 6 to 14 carbon atoms optionally having a substituent, an aralkyl group with 7 to 20 carbon atoms optionally having a substituent, or a hydroxy group;
   R¹ to R⁵, R⁸ and R⁹ can form a cyclic structure by binding to each other; and
   X⁻ represents an anion).
[7] The vinamidinium salt according to any one of the above [1] to [6], wherein X⁻ is an anion selected from the group consisting of PF₆⁻, BF₄⁻, halide ion, R^{A}CO₂ (R^{A} represents an organic group with 1 to 20 carbon atoms), and R^{B}SO₄⁻ (R^{B} represents an organic group with 1 to 20 carbon atoms).
[8] An UV-A absorbent comprising the vinamidinium salt according to any one of the above [1] to [6].
[9] The UV-A absorbent according to the above [8], wherein the UV-A absorbent has transparency.
[10] An external preparation for skin comprising the vinamidinium salt according to any one of the above [1] to [6].
[11] A glass material comprising the vinamidinium salt according to any one of the above [1] to [6].
[12] A resin material comprising the vinamidinium salt according to any one of the above [1] to [6].

### ADVANTAGEOUS EFFECT OF INVENTION

The novel vinamidinium salt of the present invention exerts an excellent UV-A absorbing effect.

### BRIEF DESCRIPTION OF THE DRAWINGS

[Figure 1] It shows the UV absorbing spectrum of vinamidinium salt (3a) of the present invention.
[Figure 2] It shows the UV absorbing spectrum of vinamidinium salt (3b) of the present invention.
[Figure 3] It shows the UV absorbing spectrum of vinamidinium salt (3c) of the present invention.
[Figure 4] It shows the UV absorbing spectrum of vinamidinium salt (3d) of the present invention.
[Figure 5] It shows the UV absorbing spectrum of vinamidinium salt (3e) of the present invention.
[Figure 6] It shows the UV absorbing spectrum of vinamidinium salt (3f) of the present invention.
[Figure 7] It shows the UV absorbing spectrum of vinamidinium salt (3g) of the present invention.
[Figure 8] It shows the UV absorbing spectrum of vinamidinium salt (3h) of the present invention.
[Figure 9] It shows the UV absorbing spectrum of vinamidinium salt (3i) of the present invention.
[Figure 10] It shows the UV absorbing spectrum of vinamidinium salt (3j) of the present invention.
[Figure 11] It shows the UV absorbing spectrum of vinamidinium salt (3k) of the present invention.
[Figure 12] It shows the UV absorbing spectrum of vinamidinium salt (3l) of the present invention.
[Figure 13] It shows the UV absorbing spectrum of vinamidinium salt (3m) of the present invention.
[Figure 14] It shows the UV absorbing spectrum of vinamidinium salt (3n) of the present invention.
[Figure 15] It shows the UV absorbing spectrum of vinamidinium salt (3o) of the present invention.
[Figure 16] It shows the UV absorbing spectrum of vinamidinium salt (7a) of the present invention.
[Figure 17] It shows the UV absorbing spectrum of vinamidinium salt (11a) of the present invention.

### DESCRIPTION OF EMBODIMENTS

The vinamidinium salt of the present invention is characterized by being shown by the following formula (1).

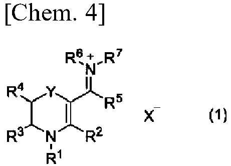

In formula (1), R¹, R², R⁵, R⁶ and R⁷ each independently represents a hydrogen atom, an alkyl group with 1 to 10 carbon atoms optionally having a substituent, an alkenyl group with 1 to 10 carbon atoms optionally having a substituent, an alkynyl group with 1 to 10 carbon atoms optionally having a substituent, an aryl group with 6 to 14 carbon atoms optionally having a substituent, an aralkyl group with 7 to 20 carbon atoms optionally having a substituent, or a hydroxy group.

As substituent, an alkoxy group with 1 to 4 carbon atoms, a halogen atom, a hydroxyl group, etc. can be exemplified. Further, as substituent of aromatic ring aryl group or aralkyl group, an alkyl group with 1 to 4 carbon atoms can be exemplified additionally to the above-mentioned substituents. As alkyl group having a substituent, a perfluoroalkyl group can be suitably exemplified.

As R¹, an alkyl with 1 to 4 carbon atoms, an alkenyl group with 1 to 4 carbon atoms, an alkynyl group with 1 to 4 carbon atoms are preferable, and an alkyl group with 1 to 4 carbon atoms is more preferable. As alkyl group with 1 to 4 carbon atoms, a methyl group, an ethyl group, a propyl group, a butyl group, an isopropyl group, etc. can be exemplified. Further, as aryl group with 6 to 14 carbon atoms, a phenyl group, a naphthyl group, etc. can be exemplified. As aralkyl group with 7 to 20 carbon atoms, a benzyl group, etc. can be exemplified.

Alkyl group, alkenyl group, alkynyl group can be linear, branched, cyclic (cycloalkyl). As alkenyl group, for example, a vinyl group, an allyl group can be exemplified. As alkynyl group, for example, an ethynyl group, a propargyl group can be exemplified.

As R², an alkyl with 1 to 4 carbon atoms, an alkenyl group with 1 to 4 carbon atoms, an alkynyl group with 1 to 4 carbon atoms are preferable, and an alkyl group with 1 to 4 carbon atoms is more preferable, and a methyl group is particularly preferable.

R³ and R⁴ each independently represents a hydrogen atom, an alkyl group with 1 to 10 carbon atoms optionally having a substituent, an alkenyl group with 1 to 10 carbon atoms optionally having a substituent, an alkynyl group with 1 to 10 carbon atoms optionally having a substituent, an alkoxy group with 1 to 10 carbon atoms optionally having a substituent, a hydroxy group or a halogen atom.

As R³ and R⁴, a hydrogen atom, an alkyl group with 1 to 4 carbon atoms, an alkenyl group with 1 to 4 carbon atoms, an alkynyl group with 1 to 4 carbon atoms are preferable, and a hydrogen atom, an alkyl group with 1 to 4 carbon atoms are more preferable, and a hydrogen atom is particularly preferable.

As R⁵, a hydrogen atom, an alkyl group with 1 to 4 carbon atoms, an alkenyl group with 1 to 4 carbon atoms, an alkynyl group with 1 to 4 carbon atoms are preferable, and a hydrogen atom, an alkyl group with 1 to 4 carbon atoms, are more preferable, and a hydrogen atom is particularly preferable.

As R⁶ and R⁷, a hydrogen atom, an alkyl group with 1 to 4 carbon atoms, a cyclic structure as described in the following are preferable.

R¹ to R⁹ can form a cyclic structure by binding to each other, and various embodiments including an embodiment in which any one of R¹ to R⁴ are bonded to each other to form a ring, an embodiment in which R² and R⁵ are bonded to each other to form a ring, an embodiment in which R⁵ to R⁷ are bonded to each other to form a ring, an embodiment in which R⁶ and R⁷ are bonded to each other to form a ring, or the like can be exemplified.

In an embodiment in which any one of R¹ to R⁴ are bonded to each other to form a ring, an embodiment in which R³ and R⁴ are bonded to each other to form a ring is preferable.

Further, in particular, R⁶ and R⁷ are preferably bonded to each other to form a ring, and specifically, it is preferable to constitute a nitrogen-containing cyclic group together with a nitrogen atom to which they are bonded.

As nitrogen-containing cyclic group, for example, azetidine group, pyrrolidine group, piperidine group, azepane group (perhydroazepine group), morpholine group, thiomorpholine group, thiomorpholine dioxide group, piperazine group, etc. can be exemplified.

Specifically, as nitrogen-containing group constituted together with a nitrogen atom to which R⁶ and R⁷ are bonded, those shown in the following can be exemplified.

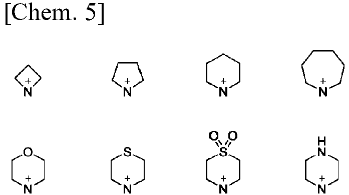

Y is O, S, NR⁸, CR⁸R⁹; R⁸ and R⁹ each independently represents a hydrogen atom, an alkyl group with 1 to 10 carbon atoms optionally having a substituent, an alkenyl group with 1 to 10 carbon atoms optionally having a substituent, an alkynyl group with 1 to 10 carbon atoms optionally having a substituent, an alkoxy group with 1 to 10 carbon atoms optionally having a substituent, an aryl group with 6 to 14 carbon atoms optionally having a substituent, an aralkyl group with 7 to 20 carbon atoms optionally having a substituent, or a hydroxy group. As Y, O (oxygen atom) or S (sulfur atom) is preferable, and O (oxygen atom) is more preferable.

X⁻ represents an anion. As anion of X⁻, PF₆⁻, BF₄⁻, a halide ion, R^{A}CO₂⁻, R^{B}SO₄⁻, etc. can be exemplified. R^{A} and R^{B} represent an organic group with 1 to 20 carbon atoms, and for example, an alkyl group with 1 to 20 carbon atoms can be exemplified, and an alkyl group with 1 to 4 carbon atoms is preferable. Among these, PF₆⁻ is particularly preferable.

As vinamidinium salt shown by formula (1) of the present invention, among these, a vinamidinium salt shown by the following formula (1A) is preferable. Specifically, in formula (1), a structure in which Y represents O, R⁶ and R⁷ constitute a morpholine group together with a nitrogen atom to which R⁶ and R⁷ are bonded is preferable.

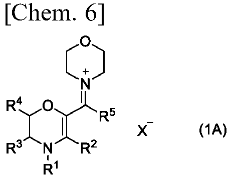

Further, in a vinamidinium salt shown by formula (1A), a vinamidinium salt in which R¹ represents an alkyl group with 1 to 4 carbon atoms, R² represents a methyl group, R³ to R⁵ represent a hydrogen atom, X⁻ represents PF₆⁻ is particularly preferable.

Further, in a vinamidinium salt shown by formula (1A), a vinamidinium salt in which R¹ represents an alkyl group with 1 to 4 carbon atoms, R² represents a methyl group, R³ and R⁴ is a cyclic structure being bonded to each other, R⁵ represent a hydrogen atom, X⁻ represents PF₆⁻ is particularly preferable.

Further, in a vinamidinium salt shown by formula (1), among these, a vinamidinium salt shown by the following formula (1B) is preferable. Specifically, in formula (1), a structure in which Y represents S, R⁶ and R⁷ constitute a morpholine group together with a nitrogen atom to which R⁶ and R⁷ are bonded is preferable.

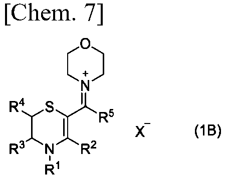

Further, in a vinamidinium salt shown by formula (1B), a vinamidinium salt in which R¹ represents an alkyl group with 1 to 4 carbon atoms, R² represents a methyl group, R³ to R⁵ represent a hydrogen atom, X⁻ represents PF₆⁻ is particularly preferable.

Other vinamidinium salts of the present invention are characterized by being shown by the following formula (2).

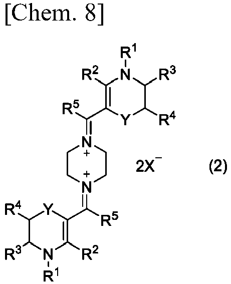

The details of R¹ to R⁵ in formula (2) are the same as explained in the above-mentioned formula (1).

As a vinamidinium salt shown by formula (2), a vinamidinium salt shown by the following formula (2A) is particularly preferable. Specifically, those in which in formula (2), Y represents O, R² represents a methyl group, R³ to R⁵ represent a hydrogen atom are particularly preferable.

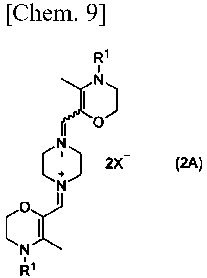

The vinamidinium salt shown by formulae (1) and (2) of the present invention absorbs light having a wavelength of UV-A region (320-400 nm). Specifically, the vinamidinium salt of the present invention has an absorption maximum wavelength (λₘₐₓ) of 320 to 400 nm.

As vinamidinium salt of the present invention, those having a molar extinction coefficient (ε) in the wavelength of UV-A region of 20000 M⁻¹ cm⁻¹ or more are preferable, those having 35000 M⁻¹ cm⁻¹ or more are more preferable, and those having 40000 M⁻¹ cm⁻¹ or more are further preferable.

The vinamidinium salt of the present invention has an excellent absorption property, even as compared to conventional compounds showing UV-A absorption property. Specifically, in the vinamidinium salt of the present invention, the maximum absorption wavelength is positioned at the long wavelength side as compared to conventional compounds showing UV-A absorption property, and can cover a wide range of UV-A region. Further, it has a high molar extinction coefficient. Further, it can absorb UV-A not pH dependently, but maintaining a stable ionic state.

Further, as vinamidinium salt of the present invention, those that do not absorb wavelength of visible light are particularly preferable. Specifically, the vinamidinium salt of the present invention is particularly preferable to have transparency.

Since the vinamidinium salt of the present invention can absorb light having wavelength in the UV-A region as in the above, it is useful as an UV-A absorbent. By utilizing the UV-A absorbing function, it can be used by mixing into external preparation to skin, glass material, resin material, etc.

Specifically, the vinamidinium salt of the present invention is useful for use in cosmetics, medicine, external preparation for skin as quasi-drug, building material, glass material and resin material as vehicle material, or general glass, plastic products, etc. Particularly, in case the vinamidinium salt of the present invention is non-benzenoid, since it is hypoallergic to skin, it is useful as external preparation for skin.

Next, one example of a method for producing vinamidinium salt of the present invention is explained.

In the following, it is explained for vinamidinium salt wherein in formula (1) or (2), Y is an oxygen atom, R² is a methyl group, R³ to R⁵ are a hydrogen atom, and X⁻ is PF₆⁻. In case Y is a sulfur atom, etc., or in case R³ to R⁵ form a cyclic structure, etc. it can be similarly produced.

As starting raw material for producing the vinamidinium salt of the present invention, morpholine shown in the following can be used.

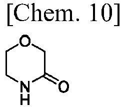

First, to nitrogen of morpholine, the starting raw material, alkyl group is introduced to obtain the following N-alkyl morpholine 1. The details of R¹ in the following N-alkyl morpholine 1 are the same as explained in the above-mentioned formula (1).

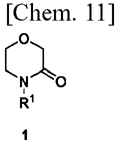

Next, the above-mentioned N-alkyl morpholine 1 is treated with methyllithium, to obtain the following cyclic enamine 2 in which the carbonyl moiety has been methylated.

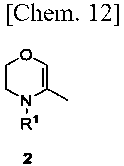

### (In case of the compound of formula (1))

Next, the above-mentioned cyclic enamine 2 is reacted with Vilsmeier-type reagent prepared from N-formylamide and phosphorus oxychloride. Next, aqueous sodium hexafluorophosphate solution is added to convert to hexafluorophosphate, and the vinamidinium salt 3 shown by formula (1) of the present invention is obtained.

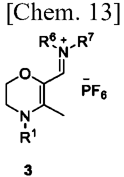

As N-formylamide in the above-mentioned reaction, specific examples include N,N-dimethylformamide, N,N-diethylformamide, N,N-diisopropylformamide, azetidine-1-carboaldehyde, pyrrolidine-1-carboaldehyde, piperidine-1-carboaldehyde, azepane-1-carboaldehyde, N,N-diphenylformamide, morpholine-4-carboaldehyde, thiomorpholine-4-carboaldehyde, thiomorpholine-4-carboaldehyde 1,1-dioxyde, etc. shown in the following.

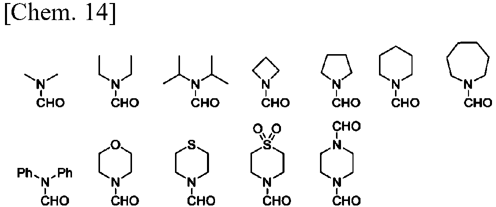

### (In case of the compound of formula (2))

For the vinamidinium salt of present invention shown by formula (2), the following piperazine-1,4-dicarboaldehyde is used as N-formylamide with respect to the cyclic enamine 2.

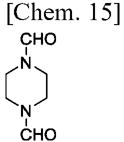

Next by adding sodium hexafluorophosphate solution to convert to hexafluorophosphate, the vinamidinium salt 3 shown by formula (2) of the present invention is obtained.

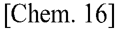

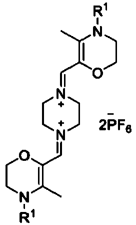

In the following, specific examples of the vinamidinium salt of the present invention are shown.

For example, in the vinamidinium salt of the present invention shown by formula (1) (the case where Y is oxygen atom is exemplified, but it can be replaced by sulfur atom, etc.), as vinamidinium salt wherein R⁶ and R⁷ are alkyl group or aryl group, the following compounds can be exemplified.

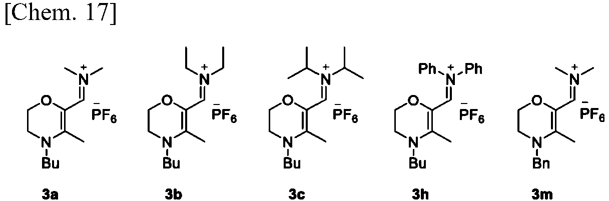

Further, for example, in the vinamidinium salt of the present invention shown by formula (1) (the case where Y is oxygen atom is exemplified, but it can be replaced by sulfur atom, etc.), as vinamidinium salt wherein R⁶ and R⁷ constitute a nitrogen-containing group together with a nitrogen atom to which R⁶ and R⁷ are bonded, the following compounds can be exemplified.

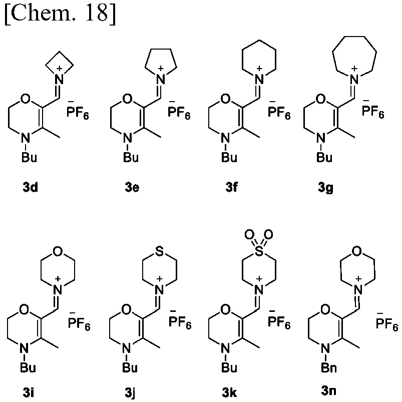

Further, for example, in the vinamidinium salt of the present invention shown by formula (1) (the case where Y is oxygen atom is exemplified, but it can be replaced by sulfur atom, etc.), as vinamidinium salt wherein R¹ is an aralkyl group, the following compounds can be exemplified.

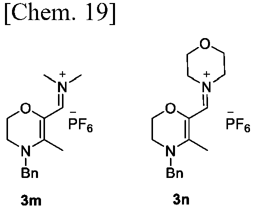

Further, for example, in the vinamidinium salt of the present invention shown by formula (1) (the case where Y is oxygen atom is exemplified, but it can be replaced by sulfur atom, etc.), as vinamidinium salt wherein R⁶ and R⁷ each are hydrogen atom, the following compounds can be exemplified.

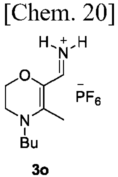

Meanwhile, vinamidinium salt 3o can be obtained by, for example by performing amine-exchange to vinamidinium salt 3a.

Further, for example, as vinamidinium salt of the present invention shown by formula (2) (the case where Y is oxygen atom is exemplified, but it can be replaced by sulfur atom, etc.), the following compounds can be exemplified.

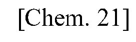

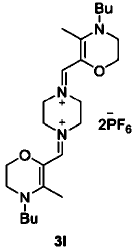

### EXAMPLES

In the following, specific examples of the present invention are explained, but the scope of the present invention is not limited to these examples.

### <Summary of the production method of the vinamidinium salt of the present invention>

As shown in the following reaction formula, first, by the alkylation reaction of morpholine, N-alkylmorpholine 1 is synthesized, and then by treating N-alkylmorpholine 1 with methyllithium, cyclic enamine 2 is prepared. Further, cyclic enamine 2 is reacted with Vilsmeier-type reagent prepared from N-formylamide and phosphorus oxychloride, and by the subsequent conversion to hexafluorophosphate, the vinamidinium salt 3 of the present invention is synthesized.

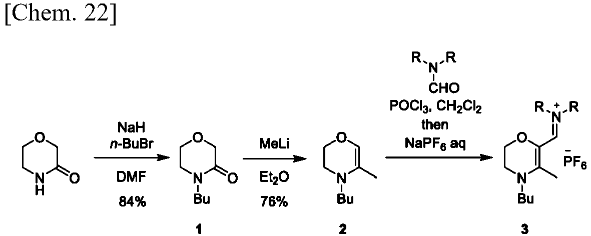

### [Example 1]

### <Production of vinamidinium salt 3a of the present invention>

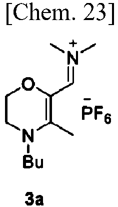

### (Synthesis of compound 1)

Under argon atmosphere, morpholine-3-one (14.9 g, 148 mmol) was dissolved in distilled and dried N,N-dimethylformamide (150 mL). The obtained solution was cooled to 0° C, added with sodium hydride (in mineral oil 60%, 7.00 g, 175 mmol). To this suspension liquid, 1-bromobutane (16.5 mL, 153 mmol) was slowly dropped, the reaction mixture was warmed to room temperature, and then stirred for 6 hours. Water was added to the reaction liquid to quench the reaction, and the mixture was extracted 3 times with ethyl acetate. The combined organic layers were washed with brine, then dried over anhydrous sodium sulfate, filtered to concentrate the filtrate. The obtained crude product was purified by reduced-pressure distillation (0.7 torr, 108 °C), to obtain 19.5 g (84%) of compound 1 (4-butylmorpholine-3-one) as a colorless transparent oil.

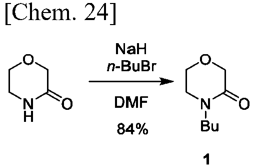

¹H NMR (400 MHz, CDCl₃) δ 4.16 (s, 2H), 3.88 (dd, *J* = 5.5, 4.6 Hz, 2H), 3.41 (t, *J* = 7.3Hz, 2H), 3.36 (apparentt, J = 5.1Hz, 2H), 1.59-1.52 (m, 2H), 1.39-1.32 (m, 2 H), 0.94 (t, *J* = 7.3Hz, 3H); ¹³C NMR (150 MHz, CDCl₃) δ 166.5, 68.1, 63.9, 46.1, 46.0, 28.9, 20.0, 13.8; IR(NaCl): 2958, 1639cm⁻¹; MS(EI) m/z (%) 157 (M⁺,28), 86 (1 00); HRMS(EI) calcdfor C₈H₁₅NO₂: 157.1103, found:157.1101.

### (Synthesis of compound 2)

Under argon atmosphere, compound 1 (3.67 g, 23.4 mmol) was dissolved in dehydrated diethyl ether (87 mL). The solution was cooled to -18 °C, and methyl lithium (1.14 M diethyl ether solution, 27.0 mL, 30.8 mmol) was slowly dropped. The reaction mixture was stirred at -18 °C for 2.5 hours, and then ice was added to quench the reaction. After raising the temperature to room temperature, the mixture was extracted 3 times with diethyl ether. The combined organic layers were washed with brine, then dried over anhydrous sodium sulfate, filtered to concentrate the filtrate. The obtained crude product was purified by reduced-pressure distillation (10 torr, 80 °C), to obtain 2.73 g (76%) of compound 2 (4-butyl-5-methyl-3,4-dihydro*-2H*-1,4-oxadine) as a colorless transparent oil.

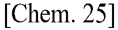

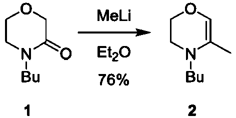

¹H NMR (400 MHz, CDCl₃) δ 5.74 (s, 1H), 3.89 (t, *J* = 4.8Hz, 2H), 3.07 (t, *J* = 4. 8 Hz, 2H), 2.78 (dd, *J* = 7.6, 7.6 Hz, 2H), 1.64 (s,3H), 1.48-1.38 (m, 2H), 1.36-1.26 (m, 2H); ¹³C NMR (100 MHz, CDCl₃) δ 123.5, 121.9, 63.0, 50.8, 47.5, 29.4, 20.2, 1 5.7, 14.0; IR(NaCl): 2958, 1607cm⁻¹: MS(EI) m/z (%) 310 (2M⁺, 65), 211 (100), 155 (M⁺, 67), 112 (60); HRMS (EI) calcdfor C₉H₁₇NO: 155.1310, found:155.1309.

### (Synthesis of compound 3a)

Under argon atmosphere, N,N-dimethylformamide (350 µL, 4.74 mmol) was added to a flask, cooled to 0 °C, and phosphorus oxychloride (90.0 µL, 0.968 mmol) was slowly dropped. The obtained solution was raised the temperature to room temperature, and then dichloromethane (3.0 mL) solution of compound 2 (98.1 mg, 0.645 mmol) was dropped. The reaction mixture was raised the temperature to 50 °C, stirred for 5 hours, then cooled to room temperature, and aqueous sodium hexafluorophosphate solution (3.0 M, 0.86 mL, 2.6 mmol) was added. The reaction mixture was stirred for 20 min. added with water, and extracted 3 times with dichloromethane. The combined organic layers were washed with brine, then dried over anhydrous magnesium sulfate, filtered, and the filtrate was concentrated with an evaporator. Further, the remained N,N-dimehtylformamide was distilled away by using Kugelrohr distillation apparatus, and the residue was purified by alumina-column chromatography (CHCl₃/hexane = 1:1 to 3:1) to obtain 140 mg (61%) of compound 3a (vinamidinium salt 3a of the present invention) as an orange-colored oil.

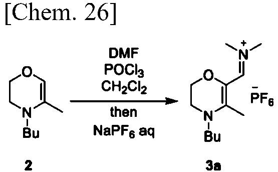

¹H NMR (400 MHz, CDCl₃ ) δ 7.22(s, 1H), 4.07 (t, *J* = 4.6Hz, 2H), 3.60 (t, *J* = 4.3 Hz, 2H), 3.51 (t, *J* = 8.0Hz, 2H), 3.41 (s, 3H), 3.31 (s, 3H), 2.30 (s, 3H), 1.66-1.62 (m, 2H), 1.39-1.30 (m, 2H), 0.97 (t, *J* = 7.3 Hz, 3H); ¹³C NMR (151 MHz, CDCl₃) δ 156.0, 145.0, 125.1, 61.7, 53.4, 48.8, 48.5, 40.2, 29.6, 19.7, 14.1, 13.5; IR(KBr): 29 62, 1641 cm⁻¹; MS(FAB⁺) m/z (%) 211 ([M-PF₆]⁺,100); MS(ESI⁻) m/z 144.9; HRMS(F AB⁺, [M-PF₆]⁺) calcdfor C₁₂H₂₃N₂O: 211.1810, found:211.1809.

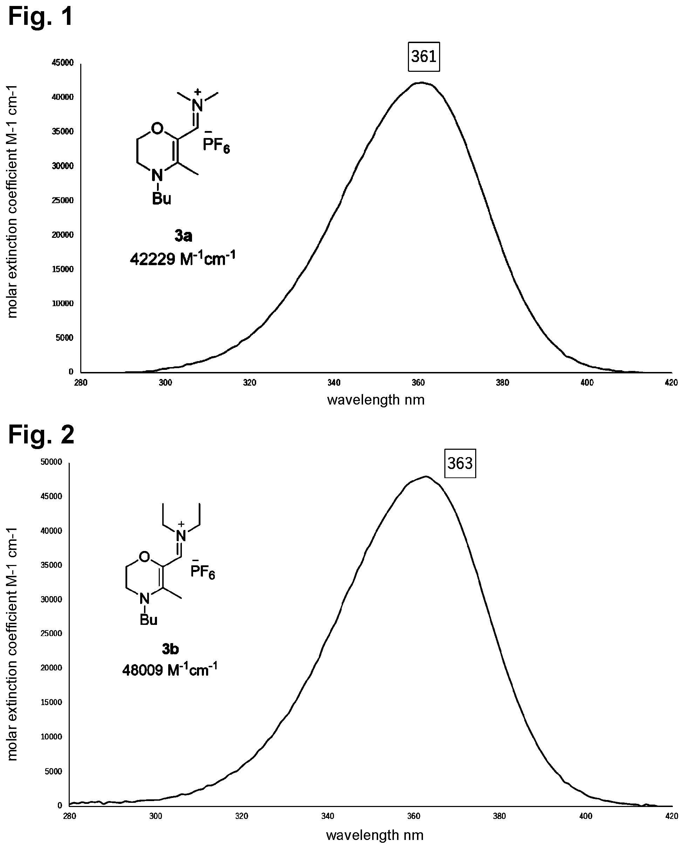

The UV absorbing spectrum (λₘₐₓ: 361 nm, ε: 42229 M⁻¹cm⁻¹) of vinamidinium salt 3a of the present invention produced in the above is shown in Fig. 1.

### [Example 2]

### <Production of vinamidinium salt 3b of the present invention>

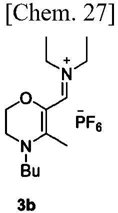

Similarly as Example 1, compound 3b (vinamidinium salt 3b of the present invention) was synthesized from compound 2 (101 mg, 0.651 mmol) and N,N-diethylformamide (520 µL, 4.68 mmol), and purified by alumina-column chromatography (CHCl₃/hexane = 1:2 to 1:1). The yield was 147 mg (59%), and was an orange-colored oil.

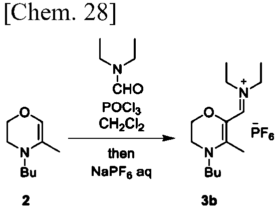

¹H NMR (400 MHz, CDCl₃) δ 7.18 (s, 1H), 4.08 (t, *J* = 4.6 Hz, 2H), 3.73 (q, *J* = 7.2 Hz, 2H), 3.63 (t, *J* = 4.3 Hz, 2H), 3.54-3.48 (m, 4H), 2.31 (s, 3H), 1.66-1.62 (m, 2H), 1.39-1.36 (m, 2H), 1.32 (t, *J* = 7.1 Hz, 4H), 1.25 (t, *J* = 7.1 Hz, 3H), 0.97 (t, *J* = 7.3 Hz, 3H); ¹³C NMR (150 MHz, CDCl₃) δ 156.2, 143.4, 124.9, 61.8, 53.9, 5 3.5, 48.9, 45.4, 29.7, 19.8, 14.7, 14.5, 14.3, 13.6; IR(KBr): 2962, 1628 cm⁻¹; MS(FA B) m/z (%) 239 ([M-PF₆]⁺, 100); HRMS(FAB) calcdfor C₁₄H₂₇N₂O [M-PF₆]⁺: 239.212 3, found: 239.2125.

The UV absorbing spectrum (λₘₐₓ: 363 nm, ε: 48009 M⁻¹cm⁻¹) of vinamidinium salt 3b of the present invention produced in the above is shown in Fig. 2.

### [Example 3]

### <Production of vinamidinium salt 3c of the present invention>

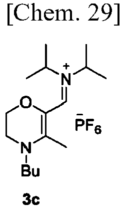

Similarly as Example 1, compound 3c (vinamidinium salt 3c of the present invention) was synthesized from compound 2 (101 mg, 0.651 mmol) and N,N-diisopropylformamide (700 µL, 4.82 mmol), and purified by alumina-column chromatography (CHCl₃/hexane = 1:10 to 1:1). The yield was 202 mg (76%), and was an orange-colored oil.

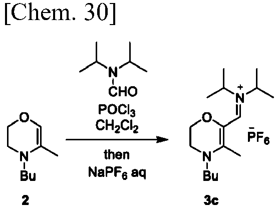

¹H NMR (600 MHz, CDCl₃) δ 7.00 (s, 1H), 5.18 (brs, 1H), 4.10 (q, *J* = 4.5 Hz, 2 H), 3.78-3.74 (m, 1H), 3.63 (t, *J* = 4.6 Hz, 2H), 3.56-3.53 (m, 2H), 2.31 (s, 3H), 1.6 6-1.61 (m, 2H), 1.38-1.34 (m, 2H), 1.32 (d, *J* = 6.9 Hz, 6H), 1.25 (d, *J* = 6.6 Hz, 6 H), 0.95 (t, *J* = 7.4 Hz, 3H); ¹³C NMR (150MHz, CDCl₃) δ 156.2, 139.1, 125.2, 61. 8, 53.7, 51.8, 49.4, 49.0, 29.6, 23.7, 20.5, 19.7, 14.6, 13.5; IR(KBr): 2968, 1622 cm⁻¹; MS(FAB) m/z (%) 267 ([M-PF₆]⁺,100); HRMS(FAB) calcdfor C₁₆H₃₁N₂O [M-PF₆]⁺: 2 67.2436, found:267.2439.

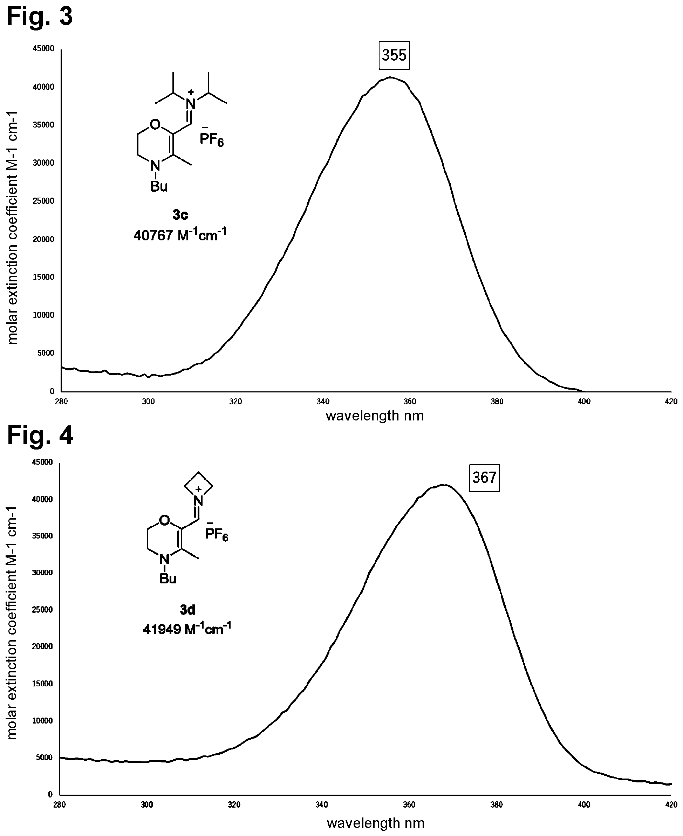

The UV absorbing spectrum (λₘₐₓ: 355 nm, ε: 40767 M⁻¹cm⁻¹) of vinamidinium salt 3c of the present invention produced in the above is shown in Fig. 3.

### [Example 4]

### <Production of vinamidinium salt 3d of the present invention>

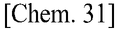

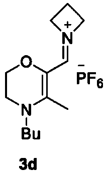

CH₂Cl₂ (42.0 mL) suspension liquid of azetidine hydrochloride (1.01 g, 10.7 mmol) was added with proton sponge (2.45 g, 11.4 mmol). The obtained suspension liquid was raised temperature to 50 °C, stirred for 1 hour, and then returned to room temperature. To a separate flask, CH₂Cl₂ (250 mL) solution of N-formylsaccharin (2.38 g, 11.2 mmol) was prepared, and amine solution prepared in the above was added thereto. The obtained reaction solution was raised temperature to 50 °C, and stirred for 23 hours. After returning to room temperature, the solvent was removed with an evaporator, and further purified by reduced pressure distillation (4.0 torr, 90 °C) by using Kugelrohr distillation apparatus to obtain 644 mg (71%) of azetidine-1-carboaldehyde as a colorless oil.

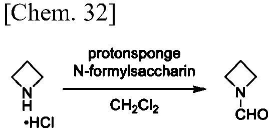

¹H NMR (400 MHz, CDCl₃) δ 7.94 (s, 1H), 4.18 (t, *J* = 7.7 Hz, 2H), 4.05 (t, *J* = 7.7 Hz, 2H), 2.42-2.34 (m, 2H)

Similarly as Example 1, compound 3d (vinamidinium salt 3d of the present invention) was synthesized from compound 2 (104 mg, 0.671 mmol) and azetidine-1-carboaldehyde (408 mg, 4.80 mmol), and purified by alumina-column chromatography (CHCl₃/hexane = 1:1). The yield was 145 mg (61%), and was a black-colored oil.

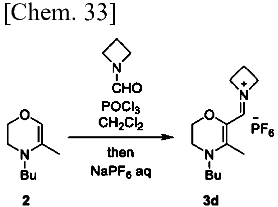

¹H NMR (600 MHz, CDCl₃) δ 7.29 (s, 1H), 4.65 (t, *J* = 7.9 Hz, 2H), 4.50 (t, *J* = 8. 0 Hz, 2H), 4.02 (t, *J* = 4.6 Hz, 2H), 3.54 (t, *J* = 4.6 Hz, 2H), 3.46 (t, *J* = 7.7 Hz, 2H), 2.49-2.43 (m, 2H), 2.22 (s, 3H), 1.64-1.59 (m, 2H), 1.39-1.32 (m, 2H), 0.97 (t, *J* = 7.3 Hz, 3H); ¹³C NMR (150 MHz, CDCl₃) δ 153.5, 143.4, 125.8, 61.9, 56.8, 56. 3, 52.9, 48.6, 29.8, 19.8, 17.2, 13.8, 13.6; IR(KBr): 2957, 1634 cm⁻¹; MS(FAB) m/z (%) 223 ([M-PF₆]⁺,100); HRMS(FAB) calcdfor C₁₃H₂₃N₂O [M-PF₆]⁺: 223.1810, found:2 23.1810.

The UV absorbing spectrum (λₘₐₓ: 367 nm, ε: 41949 M⁻¹cm⁻¹) of vinamidinium salt 3d of the present invention produced in the above is shown in Fig. 4.

### [Example 5]

### <Production of vinamidinium salt 3e of the present invention>

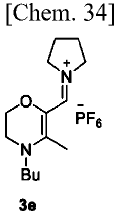

Similarly as Example 1, compound 3e (vinamidinium salt 3e of the present invention) was synthesized from compound 2 (101 mg, 0.652 mmol) and pyrrolidine-1-carboaldehyde (460 µL, 4.83 mmol), and purified by alumina-column chromatography (CHCl₃/hexane = 1:2 to 2:1). The yield was 150 mg (61%), and was an orange-colored crystal.

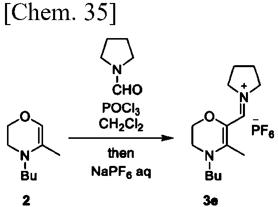

¹H NMR (600 MHz, CDCl₃) δ 7.48 (s, 1H), 4.05 (t, *J* = 4.6Hz, 2H), 3.91 (t, *J* = 6. 9 Hz, 2H), 3.78 (t, *J* = 6.6 Hz, 2H), 3.59 (t, *J* = 4.6 Hz, 2H), 3.50 (t, *J* = 7.8 Hz, 2H), 2.29 (s, 3H), 2.04-1.97 (m,2H), 1.95-1.88 (m, 2H), 1.65-1.59 (m, 2H), 1.40-1.31 (m,2H), 0.97 (t, *J* = 7.3 Hz, 3H); ¹³C NMR (150 MHz, CDCl₃) δ 155.1, 142.4, 125. 4, 61.8, 55.8, 53.1, 50.4, 48.8, 29.7, 25.8, 23.9, 19.7, 14.0, 13.5; IR(KBr): 2960, 1628 cm⁻¹; MS(FAB+) m/z (%) 237 ([M-PF₆]⁺, 100); MS(ESI⁻) m/z 144.9; HRMS(FAB) cal cd for C₁₄H₂₅N₂O [M-PF₆]⁺: 237.1967, found:237.1968.

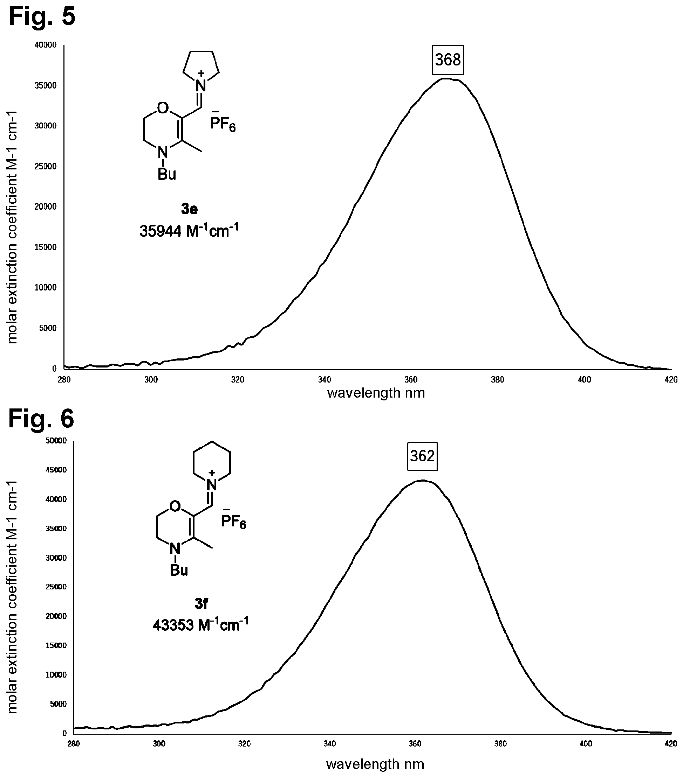

The UV absorbing spectrum (λₘₐₓ: 368 nm, ε: 35944 M⁻¹cm⁻¹) of vinamidinium salt 3e of the present invention produced in the above is shown in Fig. 5.

### [Example 6]

### <Production of vinamidinium salt 3f of the present invention>

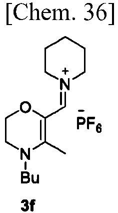

Similarly as Example 1, compound 3f (vinamidinium salt 3f of the present invention) was synthesized from compound 2 (101 mg, 0.652 mmol) and piperidine-1-carboaldehyde (520 µL, 4.69 mmol), and purified by alumina-column chromatography (CHCl₃/hexane = 1:2 to 1:1). The yield was 182 mg (71%), and was an orange-colored oil.

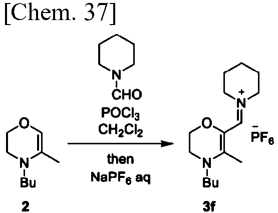

¹H NMR (400 MHz, CDCl₃) δ 7.17 (s, 1H), 4.08 (t, *J* = 4.6 Hz, 2H), 4.03 (brs, 2H), 3.62-3.57 (m, 4H), 3.51 (t, *J* = 7.8 Hz, 2H), 2.30 (s, 3H), 1.79 (brs, 2H), 1.70 (brs, 4H), 1.65-1.60 (m, 2H), 1.41-1.32 (m, 2H), 0.96 (t, *J* = 7.3 Hz, 3H); ¹³C NMR (150 MHz, CDCl₃) δ 155.8, 142.8, 124.5, 61.8, 58.7, 53.4, 49.5, 48.8, 29.7, 27.0, 26.3, 23. 6, 19.7, 14.2, 13.6; IR(KBr): 2936, 1630 cm⁻¹; MS(FAB⁺) m/z (%) 251 ([M-PF₆]⁺,10 0); MS(ESI⁻) m/z 144.9; HRMS(FAB⁺) calcd for C₁₅H₂₇N₂O [M-PF₆]⁺: 251.2123, foun d: 251.2120.

The UV absorbing spectrum (λₘₐₓ: 362 nm, ε: 43353 M⁻¹cm⁻¹) of vinamidinium salt 3f of the present invention produced in the above is shown in Fig. 6.

### [Example 7]

### <Production of vinamidinium salt 3g of the present invention>

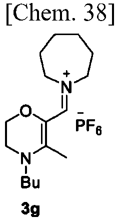

To the suspension solution of N-formylsaccharine (2.13 g, 10.1 mmol) and THF (10.0 mL), azepane (1.10 mL, 9.78 mmol) was dropped at room temperature. After stirring for 1 hour, it was diluted with dichloromethane, and added with saturated aqueous sodium bicarbonate solution. The mixture was extracted 3 times with dichloromethane, and the organic layer was washed with brine, and dried over anhydrous magnesium sulfate, filtered, and the filtrate was concentrated with an evaporator. The obtained crude product was purified by reduced pressure distillation (2.5 torr, 120 °C) to obtain 658 mg (51%) of azepane-1-carboaldehyde as a colorless oil.

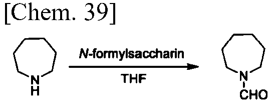

¹H NMR (400 MHz, CDCl₃) δ 8.08 (s, 1H), 3.46 (t, *J* = 5.9 Hz, 2H), 3.38 (t, *J* = 5.9 Hz, 2H), 1.76-1.72 (m, 4H), 1.60-1.56 (m, 4H)

Similarly as Example 1, compound 3g (vinamidinium salt 3g of the present invention) was synthesized from compound 2 (102 mg, 0.658 mmol) and azepane-1-carboaldehyde (629 mg, 4.95 mmol), and purified by alumina-column chromatography (CHCl₃/hexane = 1:5). The yield was 133 mg (71%), and was a black-colored oil.

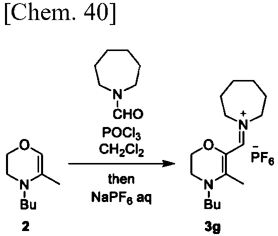

¹H NMR (400 MHz, CDCl₃) δ 7.20 (s, 1H), 4.06 (t, *J* = 4.6 Hz, 2H), 3.86 (t, *J* = 5. 9 Hz, 2H), 3.65 (t, *J* = 6.4 Hz, 2H), 3.61 (d, *J* = 4.6 Hz, 2H), 3.52 (t, *J* = 7.8 Hz, 2H), 2.32 (s, 3H), 1.84 (m, 2H), 1.77 (m, 2H), 1.67-1.61 (m, 6H), 1.42-1.32 (m, 2 H), 0.97 (t, *J* = 7.3 Hz, 3H); ¹³C NMR (150 MHz, CDCl₃) δ 156.3, 144.6, 125.1, 61. 9, 60.6, 53.6, 51.3, 49.0, 29.8, 29.0, 28.4, 26.8, 25.3, 19.8, 14.4, 13.6; IR(KBr): 2959, 1624 cm⁻¹; MS(FAB⁺) m/z (%) 265 ([M-PF₆]⁺, 100); MS(ESI⁻) m/z 144.9; HRMS(FA B⁺) calcd for C₁₆H₂₉N₂O [M-PF₆]⁺: 265.2280, found: 265.2279.

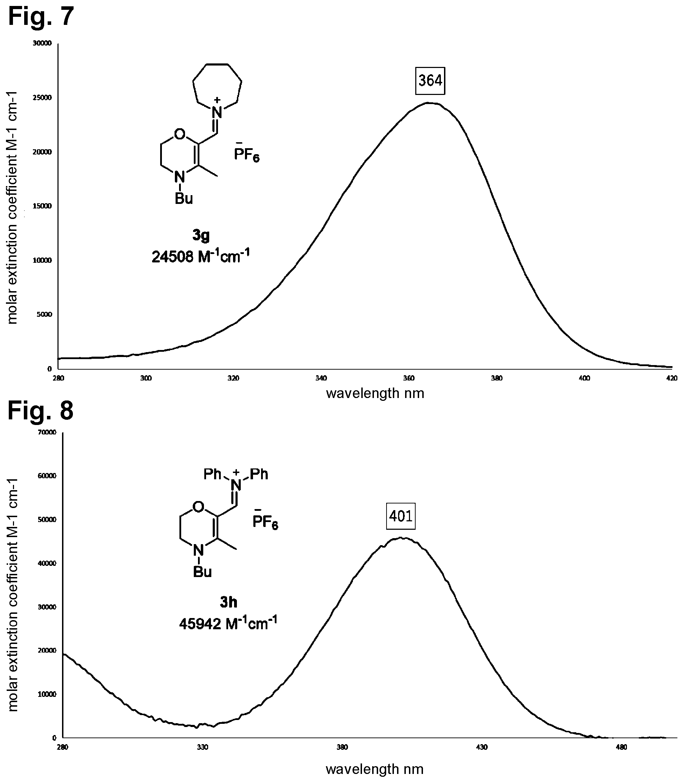

The UV absorbing spectrum (λₘₐₓ: 364 nm, ε: 24508 M⁻¹cm⁻¹) of vinamidinium salt 3g of the present invention produced in the above is shown in Fig. 7.

### [Example 8]

### <Production of vinamidinium salt 3h of the present invention>

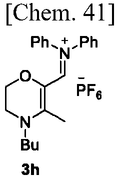

N,N-diphenylformamide (191 mg, 0.970 mmol) was dissolved in dichloromethane (360 µL), cooled to 0 °C, and phosphorus oxychloride (90.0 µL, 0.968 mmol) was slowly dropped to the solution. Then, similarly as Example 1, compound 3h (vinamidinium salt 3h of the present invention) was synthesized from compound 2 (100 mg, 0.645 mmol). After purifying by alumina-column chromatography (CHCl₃/hexane = 1:5), recrystallization (MeOH/hexane) was further performed. The yield was 31 mg (10%), and was a pale yellow-colored prism crystal.

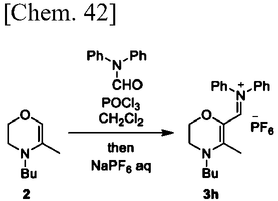

¹H NMR (400 MHz, CDCl₃) δ 7.42-7.37 (m, 5H), 7.31 (t, *J* = 7.3 Hz, 2H), 7.14 (d, *J* = 7.8 Hz, 4H), 3.75 (t, *J* = 4.8 Hz, 2H), 3.70 (t, *J* = 7.8 Hz, 2H), 3.62 (t, *J* =4. 6 Hz, 2H), 2.49 (s, 3H), 1.75-1.67 (m, 2H), 1.45-1.35 (m, 2H), 0.99 (t, *J* = 7.3 Hz, 3H); ¹³C NMR (150 MHz, CDCl₃) δ 161.7, 136.3, 129.4, 129.3, 127.3, 124.7, 60.2, 55.1, 49.6, 29.3, 19.9, 15.0, 13.6; IR(KBr): 2963, 1620, 1573 cm⁻¹; MS(FAB⁺) m/z (%) 335 ([M-PF₆]⁺, 100); MS(ESI⁻) m/z 144.9; HRMS(FAB⁺) calcd for C₂₂H₂₇N₂O [M-PF₆]⁺: 335.2123, found: 335.2124.

The UV absorbing spectrum (λₘₐₓ: 401 nm, ε: 45942 M⁻¹cm⁻¹) of vinamidinium salt 3h of the present invention produced in the above is shown in Fig. 8.

### [Example 9]

### <Production of vinamidinium salt 3i of the present invention>

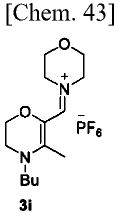

Similarly as Example 1, compound 3i (vinamidinium salt 3i of the present invention) was synthesized from compound 2 (300 mg, 1.94 mmol) and morpholine-4-carboaldehyde (1.44 mL, 14.3 mmol), and purified by alumina-column chromatography (AcOEt/hexane = 1:20 to 1:1) and recrystallization (isopropanol). The yield was 324 mg (42%), and was a colorless crystal (mp.91.2-92.2 °C).

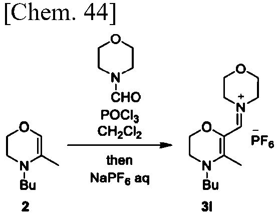

¹H NMR (600 MHz, CDCl₃) δ 7.24 (s, 1H), 4.13 (brs, 2H), 4.09 (t, *J* = 4.7 Hz, 2H), 3.85 (brs, 2H), 3.77 (brs, 2H), 3.66 (brs, 2H), 3.61 (t, *J* = 4.6 Hz, 2H), 3.53 (t, *J* = 7.8 Hz, 2H), 2.34 (s, 3H), 1.66-1.62 (m, 2H), 1.40-1.34 (m, 2H), 0.97 (t, *J* = 7.4 H z, 3H); ¹³C NMR (150 MHz, CDCl₃) δ 157.1, 142.3, 124.8, 67.0, 66.4, 61.6, 56.2, 5 3.6, 49.1, 48.8, 29.5, 19.6, 14.1, 13.4; IR(KBr): 2936, 1643, 1558 cm⁻¹; MS(FAB⁺) m/ z (%) 253 ([M-PF₆]⁺, 100); MS(ESI⁻) m/z 144.9; HRMS(FAB⁺) calcd for C₁₄H₂₅N₂O₂ [M-PF₆]⁺: 253.1916, found:253.1913.

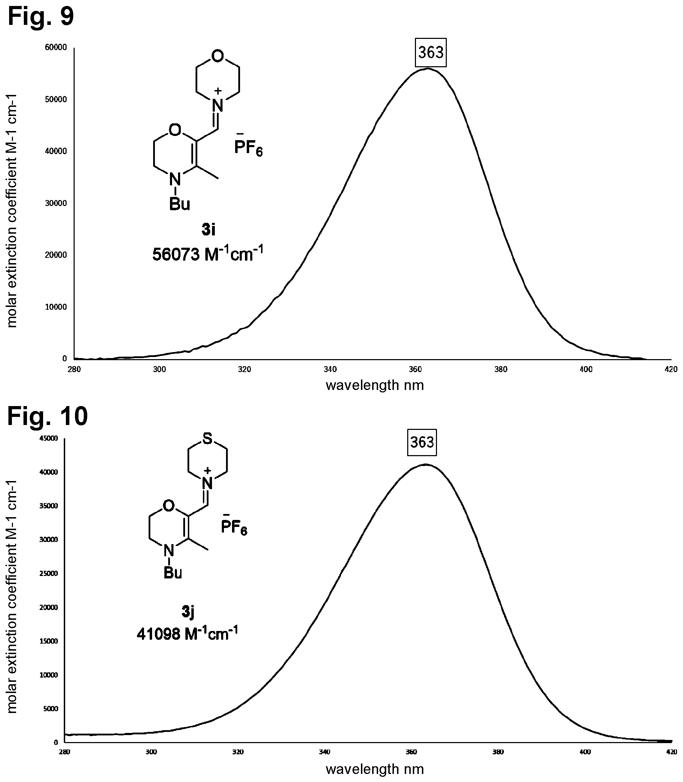

The UV absorbing spectrum (λₘₐₓ: 363 nm, ε: 56073 M⁻¹cm⁻¹) of vinamidinium salt 3i of the present invention produced in the above is shown in Fig. 9.

### [Example 10]

### <Production of vinamidinium salt 3j of the present invention>

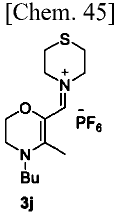

Similarly as Example 7, thiomorpholine-4-carboaldehyde was synthesized from thiomorpholine (1.46 mL, 14.6 mmol), and purified by reduced pressure distillation (1.5 torr, 150 °C). The yield was 658 mg (51%) and was a colorless oil.

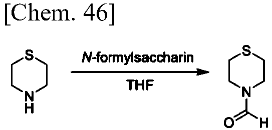

¹H NMR (400 MHz, CDCl₃) δ 8.03 (s, 1H), 3.82-3.79 (m, 2H), 3.65-3.62 (m, 2H), 2. 66-2.59 (m, 4H); ¹³C NMR (101 MHz, CDCl₃) δ 160.9, 48.1, 42.1, 28.1, 26.8; IR(KB r): 2916, 1667 cm⁻¹; MS(EI⁺) m/z (%) 131 (M⁺,100), 103(14); HRMS(EI) calcd for C₅ H₉NOS: 131.04005, found: 131.0402.

After cooling thiomorpholine-4-carboaldehyde (627 mg, 4.79 mmol) to 10 °C, phosphorus oxychloride (90.0 µL, 0.968 mmol) was slowly dropped. Thereafter, similarly as Example 1, compound 3j (vinamidinium salt 3j of the present invention) was synthesized from compound 2 (100 mg, 0.645 mmol). It was purified by alumina-column chromatography (AcOEt/hexane = 1:20 to 1:1), and recrystallization (isopropanol). The yield was 324 mg (42%), and was a black-colored solid.

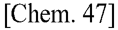

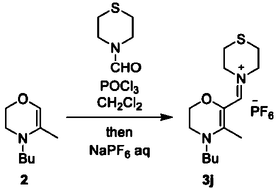

¹H NMR (400 MHz, CDCl₃) δ 7.19 (s, 1H), 4.30 (brs, 2H), 4.10 (t, *J* = 4.6 Hz, 2H), 3.87 (brs, 2H), 3.63 (t, *J* = 4.6 Hz, 2H), 3.54 (t, *J* = 7.8 Hz, 2H), 2.85 (brs, 2H), 2.75 (brs, 2H), 2.34 (s, 3H), 1.67-1.61 (m, 2H), 1.42-1.33 (m, 2H), 0.97 (t, *J* = 7.3 Hz, 3H); ¹³C NMR (150 MHz, CDCl₃) δ 157.9, 142.6, 125.0, 61.8, 59.5, 53.9, 51.4, 49.0, 29.6, 28.6, 28.1, 19.8, 14.5, 13.6; IR(KBr): 2959, 1636, 1566 cm⁻¹; MS(FAB⁺) m/z (%) 269 ([M-PF₆]⁺, 100); MS(ESI⁻) m/z 144.9; HRMS(FAB⁺) calcd for C₁₄H₂₅N₂O S [M-PF₆]⁺: 269.1688, found:269.1687.

The UV absorbing spectrum (λₘₐₓ: 363 nm, ε: 41098 M⁻¹cm⁻¹) of vinamidinium salt 3j of the present invention produced in the above is shown in Fig. 10.

### [Example 11]

Similarly as Example 7, thiomorpholine-4-carboaldehyde 1,1-dioxide was synthesized from thiomorpholine-1,1-dioxide (1.46 g, 10.8 mmol), and purified by silica gel chromatography (CH₂Cl₂/MeOH = 20:1). The yield was 908 mg (50%), as was a colorless prism.

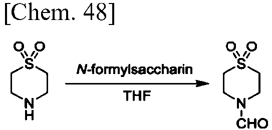

¹H NMR (400 MHz, CDCl₃) δ 8.11 (s, 1H), 4.05 (t, *J* = 5.5 Hz, 2H), 3.89 (t, *J* = 5. 5 Hz, 2H), 3.10-3.04 (m, 4H); ¹³C NMR (100 MHz, CDCl₃) δ 160.8, 52.3, 51.4, 43. 8, 37.7; IR(KBr): 2937, 1665 cm⁻¹; MS(EI⁺) m/z (%) 163 (M⁺,32), 99 (100) ;MS(ESI ⁻) m/z 144.9; HRMS(EI⁺) calcd for C₅H₉NO₃S: 163.0298, found:163.0301.

Thiomorpholine-4-carboaldehyde 1,1-dioxide (158 mg, 0.969 mmol) was dissolved in acetonitrile (360 µL), cooled to 20 °C, and then phosphorus oxychloride (90.0 µL, 0.968 mmol) was slowly dropped to this solution. The obtained solution was raised temperature to room temperature, and then acetonitrile (0.30 mL) solution of compound 2 (100 mg, 0.645 mmol) was dropped. Thereafter, similarly as Example 1, compound 3k (vinamidinium salt 3k of the present invention) was synthesized from purification by resuspension (MeOH/CHCl₃). The yield was 17.9 mg (6%) and was yellow-colored prism.

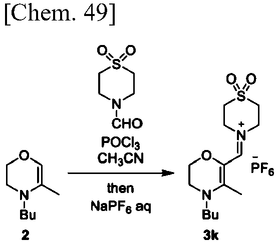

¹H NMR (600 MHz, DMSO-d₆, 100 °C) δ 7.44 (s, 1H), 4.19 (brs, 4H),4.08 (t, *J* = 4. 8 Hz, 2H), 3.71 (t, *J* = 4.5 Hz, 2H), 3.63 (t, *J* = 7.6 Hz, 2H), 3.33 (brs, 4H), 2.37 (s, 3H), 1.68-1.63 (m, 2H), 1.39-1.33 (m, 2H), 0.94 (t, *J* = 7.2 Hz, 3H); ¹³C NMR (150 MHz, DMSO-d₆,100 °C) δ 159.7, 141.9, 125.2, 61.4, 53.1, 51.5, 49.6, 48.4, 28.4, 18.7, 13.9, 12.8; IR(KBr): 2961, 1636, 1562 cm⁻¹; MS(FAB⁺) m/z (%) 301 ([M-PF₆]⁺, 100); HRMS(FAB⁺) calcd for C₁₄H₂₅N₂O₃S [M-PF₆]⁺: 301.1586, found: 301.1586.

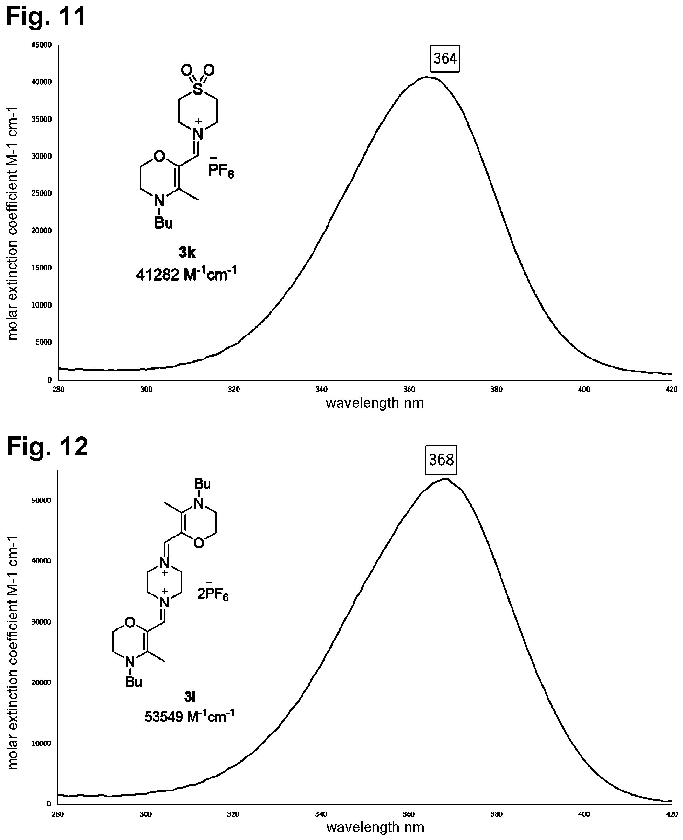

The UV absorbing spectrum (λₘₐₓ: 364 nm, ε: 41282 M⁻¹cm⁻¹) of vinamidinium salt 3k of the present invention produced in the above is shown in Fig. 11.

### [Example 12]

### <Production of vinamidinium salt 3l of the present invention>

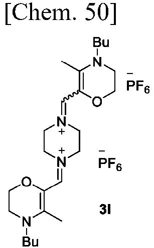

Similarly as Example 1, compound 3l (vinamidinium salt 3l of the present invention (EZ isomer mixture, 2.3:1)) was synthesized from compound 2 (149 mg, 0.961 mmol) and piperadine-1,4-carboaldehyde (45.9 mg, 0.323 mmol), and purified by recrystallization (methanol). The yield was 79.9 mg (35%), and was a yellow green-colored crystal.

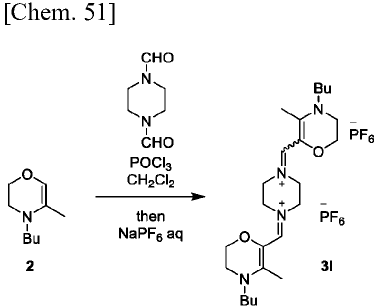

¹H NMR (400 MHz, DMSO-d₆) δ 7.53 (s, 1.4H), 7.52 (s, 0.6H), 4.07-4.35 (brs, 3H), 4.04 (t, *J =* 4.1 Hz, 4H), 4.02-3.69 (br, 6H), 3.68 (t, *J =* 4.1 Hz, 4H), 3.59 (t, *J =* 7.5 Hz, 4H), 2.35 (s, 4.2H), 2.34 (s, 1.8H), 1.63-1.58 (m, 4H), 1.32 (q, *J =* 7.5 H z, 4H), 0.93 (t, *J =* 7.3Hz, 6H); ¹³C NMR (150 MHz, DMSO-d₆) δ 158.1, 142.1, 12 4.4, 61.7, 54.7, 53.1, 48.5, 48.0, 28.9, 19.3, 14.1, 13.6; IR(KBr): 2959, 1692, 1635, 1570 cm⁻¹; MS(FAB⁺) m/z (%) 563 ([M-PF₆]⁺, 100); HRMS(FAB⁺) calced for C₂₄H₄₂ F₆N₄O₂P [M-PF₆]⁺: 563.2950, found:563.2946.

The UV absorbing spectrum (λₘₐₓ: 368 nm, ε: 53549 M⁻¹cm⁻¹) of vinamidinium salt 31 of the present invention produced in the above is shown in Fig. 12.

### [Example 13]

### <Production of vinamidinium salt 3m of the present invention>

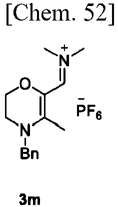

DMF (0.300 mL, 3.88 mmol) cooled to 0 °C was added with POCl₃ (75.0 µL, 0.804 mmol) and raised temperature to room temperature. Then, CH₂Cl₂ (0.110 mL) solution of benzyl enamine (100 mg, 0.529 mmol) was added, and then raised temperature to 50°C. After stirring for 5 hours, it was returned to room temperature, then 5 M NaPF₆ aqueous solution (160 µL, 0.800 mmol) was added and stirred. Then, it was extracted with CH₂Cl₂, the organic layer was washed with water, and dried over MgSO₄. The obtained crude product was purified by alumina-column (CHCl₃/MeOH = 40: 1) and the intended product being red-colored crystal was obtained at 149 mg (74%).

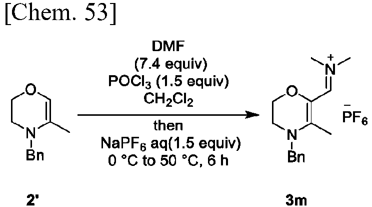

¹H NMR (400 MHz, CDCl₃) δ 7.37 (t, *J* = 7.3 Hz, 2H), 7.30 (t, *J* = 7.5Hz, 1H), 7.27 (s, 1H), 7.15 (d, *J=* 7.3 Hz, 2H), 4.72 (s, 2H), 4.04 (t, *J=* 4.6 Hz, 2H), 3.54 (t, *J= 4.6* Hz, 2H), 3.41 (s, 3H), 3.29 (s, 3H), 2.30 (s, 3H).

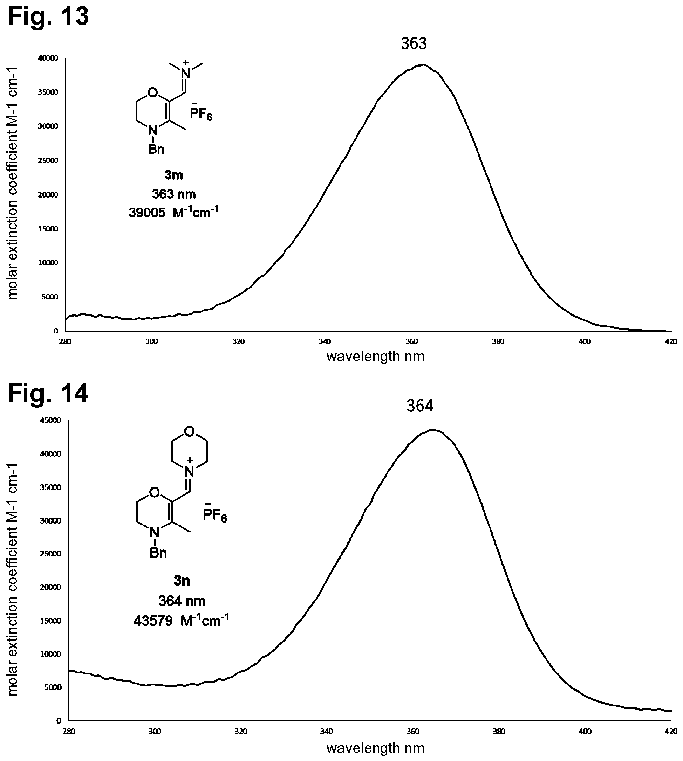

The UV absorbing spectrum (λₘₐₓ: 363 nm, ε: 39005 M⁻¹cm⁻¹) of vinamidinium salt 3i of the present invention produced in the above is shown in Fig. 13.

### [Example 14]

### <Production of vinamidinium salt 3n of the present invention>

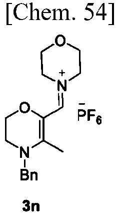

N-Formylmorpholine (0.390 mL, 3.87 mmol) cooled to 18 °C was added with POCl₃ (75.0 µL, 0.804 mmol) and raised temperature to room temperature. Thereto, CH₂Cl₂ (0.110 mL) solution of benzyl enamine (103 mg, 0.547 mmol) and the temperature was raised to 50 °C. After stirring for 5 hours, it was returned to room temperature, and 5 M NaPH₆ aqueous solution (160 µL, 0.800 mmol) was added and stirred. Then, it was extracted with CH₂Cl₂, the organic layer was washed with water, and dried over MgSO₄. By adding CHCl₃ to the obtained crude product in oil paste, the intended product was precipitated. The crystal was separated and the intended product being a pale yellow-colored prism crystal was obtained at 163 mg (72%).

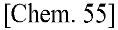

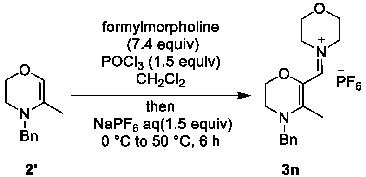

¹H NMR (400 MHz, DMSO-d₆) δ 7.65 (s, 1H), 7.43 (dd, *J =* 8.0, 6.6 Hz, 2H), 7.37 (d, *J =* 7.3 Hz, 1H), 7.28 (d, *J =* 6.9 Hz, 2H), 4.89 (s, 2H), 4.11 (s, 2H), 4.08 (t, *J =* 4.6 Hz, 2H), 3.74 (s, 2H), 3.70 (s, 4H), 3.62 (t, *J =* 4.6 Hz, 2H), 2.36 (s, 3H). MS(ESI⁺) m/z 287.1 (M-PF₆); (ESI⁻) m/z 144.9.

The UV absorbing spectrum (λₘₐₓ: 364 nm, ε: 43579 M⁻¹cm⁻¹) of vinamidinium salt 3n of the present invention produced in the above is shown in Fig. 14.

### [Example 15]

### <Production of vinamidinium salt 3o of the present invention>

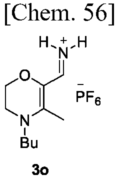

Vinamidinium salt 3a (10.2 mg, 0.0285 mmol) was dissolved in ammonia methanol solution (7 M, 400 µL, 2.80 mmol) and stirred at room temperature for 2 hours. The reaction solution was concentrated under reduced pressure with an evaporator, the obtained crude product was recrystallized from MeOH/CHCl₃, and the intended product (vinamidinium salt 3o of the present invention) being a colorless and transparent prism was obtained at 9.1 mg (99%).

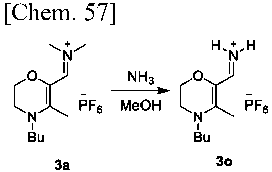

mp: >124 °C (decomp); ¹H NMR (400 MHz, CD₃OD) δ 7.88 (s, 1H), 4.11 (t, *J* = 4. 3Hz, 2H), 3.55-3.48(m, 4H), 2.23 (s, 3H), 1.72-1.64 (m, 2H), 1.41-1.31 (m, 2H), 0.98 (t, *J* = 7.3 Hz, 3H); ¹³C NMR (150 MHz, CD₃OD) δ 161.8, 143.1, 126.1, 63.8, 57.8, 47.0, 31.0, 20.6, 14.9, 13.9; IR(KBr): 2962, 1612 cm⁻¹; MS(EI) m/z (%) 183 ([M-PF ₆]⁺, 100); HRMS(EI) calced for C₁₀H₁₉N₂O [M-PF₆]⁺: 183.1497, found:183.1497.

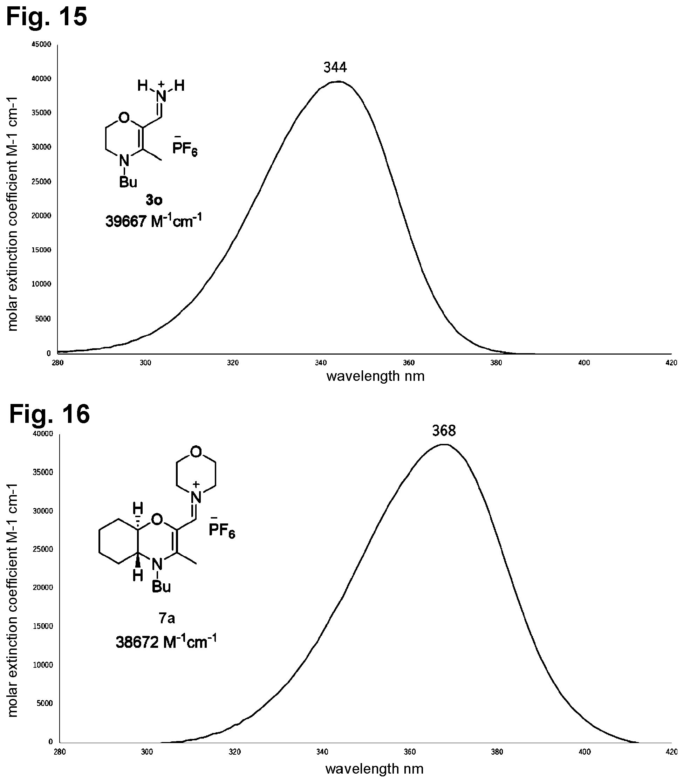

The UV absorbing spectrum (solvent: MeOH, λₘₐₓ: 344 nm, ε: 39667 M⁻¹cm⁻¹) of vinamidinium salt 3o of the present invention produced in the above is shown in Fig. 15.

### [Example 16]

### <Production of vinamidinium salt 7a of the present invention>

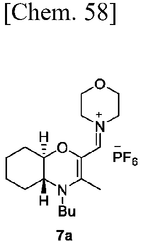

### <Summary of the production method>

As shown in the following reaction formula, first, by the alkylation reaction of compound 4 having a morpholine structure, compound 5 having a N-alkylmorpholine structure is synthesized. Then, by treating compound 5 with methyllithium, compound 6 having a cyclic enamine structure is prepared. Further, compound 6 is reacted with Vilsmeier-type reagent prepared from N-formylamide and phosphorus oxychloride, and by the subsequent conversion to hexafluorophosphate, the vinamidinium salt 7a of the present invention is synthesized.

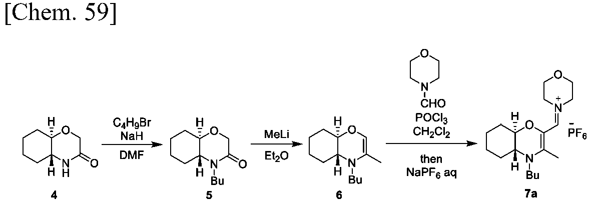

### <Specific production method>

### (Synthesis of compound 5)

Compound 4 (503 mg, 3.24 mmol) prepared according to the literature (S.Dugar, A.Sharma, B.Kuila, D.Mahajan, S.Dwivedi, VTripathi, Synthesis, 2015,47,712.) was dissolved in distilled and dried N,N-dimethylformamide (6.30 mL). The obtained solution was cooled to 18 °C, added with sodium hydride (in mineral oil 60%, 221 g, 5.53 mmol). To this suspension liquid, 1-bromobutane (560 µL, 5.19 mmol) was slowly dropped, the reaction mixture was warmed to room temperature, and then stirred for 18 hours. Water was added to the reaction liquid to quench the reaction, and the mixture was extracted 3 times with ethyl acetate. The combined organic layers were washed with brine, then dried over anhydrous sodium sulfate, filtered to concentrate the filtrate. The obtained crude product was purified by silica gel column chromatography (AcOEt/hexane = 1:5) and reduced-pressure distillation (0.14 torr, 180 °C), to obtain 587 g (86%) of compound 5 (4-butylhexahydro-2H-benzo [b][1,4]oxadine-3(4H)-one) as a colorless transparent oil.

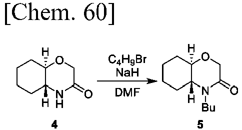

¹H NMR (400 MHz, CDCl₃) δ 4.29 (d, *J* = 16.4 Hz, 1H), 4.21 (d, *J* = 16.4 Hz, 1 H), 3.78 (ddd, *J* = 15.1, 8.6, 5.2 Hz, 1H), 3.31-3.27 (m, 1H), 3.19-3.11 (m, 2H), 2.2 0-2.17 (m, 1H), 2.04-2.01 (m, 1H), 1.84-1.81 (m, 2H), 1.58-1.54 (m, 1H), 1.44-1.27 (m, 6H), 1.21-1.16 (m, 1H), 0.93 (t, *J* = 7.3 Hz, 3H); ¹³C NMR (151 MHz, CDCl₃) δ 167.3, 78.1, 68.3, 58.8, 40.4, 30.6, 29.7, 28.5, 23.9, 23.7, 20.0, 13.7; IR(KBr): 2939, 1651 cm⁻¹.

### (Synthesis of compound 6)

Under argon atmosphere, compound 5 (302 mg, 1.43 mmol) was dissolved in dehydrated diethyl ether (5.20 mL). The solution was cooled to -18 °C, and methyl lithium (1.07 M diethyl ether solution, 1.70 mL, 1.82 mmol) was slowly dropped. The reaction mixture was stirred at -18 °C for 8 hours, and ice was added to quench the reaction. After raising the temperature to room temperature, the mixture was extracted 3 times with diethyl ether. The combined organic layers were washed with brine, then dried over anhydrous sodium sulfate, filtered to concentrate the filtrate. The crude product was purified by reduced-pressure distillation (1.6 torr, 100 °C), to obtain 159 mg (54%) of compound 6 (4-butyl-3-methyl-4a,5,6,7,8,8a-hexahydro-4H-benzo[b][1,4]oxadine) as a pale orange-colored oil.

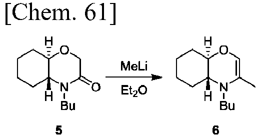

¹H NMR (400 MHz, CDCl₃) δ 5.70 (d, *J* = 1.4 Hz, 1H), 3.53-3.48 (m, 1H), 2.97-2.9 2 (m, 2H), 2.79-2.73 (m, 1H), 2.22-2.19 (m, 1H), 2.04-1.97 (m, 1H), 1.78-1.75 (m, 2 H), 1.65 (d, *J* = 0.9 Hz, 3H), 1.51-1.45 (m, 1H), 1.32-1.18 (m, 6H), 1.11-1.07 (m, 1 H), 0.92 (t, *J* = 7.3 Hz, 3H); ¹³C NMR (150 MHz, CDCl₃) δ 122.8, 122.7, 76.9, 57. 8, 44.3, 31.2, 28.2, 27.6, 24.4, 24.0, 20.3, 15.5, 14.0; IR(KBr): 2934, 1667 cm⁻¹.

### (Synthesis of compound 7a)

Under argon atmosphere, N-formylmorpholine (350 µL, 3.47 mmol) was added to a flask, cooled to 13 °C, and phosphorus oxychloride (68.0 µL, 0.729 mmol) was slowly dropped. The obtained solution was raised the temperature to room temperature, and then dichloromethane (200 µL) solution of compound 6 (101 mg, 0.483 mmol) was dropped. The reaction mixture was raised the temperature to 50 °C, stirred for 4 hours, then cooled to room temperature, and aqueous sodium hexafluorophosphate solution (3.0 M, 650 µL, 1.95 mmol) was added. The reaction mixture was stirred for 30 min. added with water, and extracted 3 times with dichloromethane. The combined organic layers were washed with brine, then dried over anhydrous magnesium sulfate, filtered, and the filtrate was concentrated with an evaporator. Further, the remained N-formylmorpholine was distilled away by using Kugelrohr distillation apparatus, and the residue was recrystallized from ethanol, and 124 mg (57%) of compound 7a was obtained as brown colored prism.

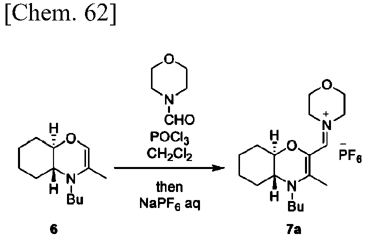

¹H NMR (400 MHz, CDCl₃) δ 7.35 (s, 1H), 4.22-4.07 (m, 2H), 3.87-3.83 (m, 2H), 3. 80-3.74 (m, 2H), 3.71-3.66 (m, 2H), 3.63-3.40 (m, 3H), 3.29-3.24 (m, 1H), 2.36 (s, 3 H), 2.35-2.28 (m, 1H), 2.17-2.10 (m, 1H), 1.95-1.86 (m, 2H), 1.56-1.30 (m, 8H), 0.97 (t, *J* = 7.3 Hz, 3H); ¹³C NMR (100 MHz, CDCl₃) δ 158.1, 143.2, 125.6, 75.7, 67.1, 66.4, 60.5, 56.4, 49.2, 46.9, 30.7, 30.5, 27.5, 24.1, 23.1, 19.7, 15.0, 13.5; IR(KBr): 29 47, 1624 cm⁻¹; MS(EI) m/z (%) 307 ([M-PF₆]⁺, 100); HRMS(EI) calced for C₁₈H₃₁N₂ O₂ [M-PF₆]⁺: 307.2386, found: 307.2386.

The UV absorbing spectrum (solvent: CHCl₃, λₘₐₓ: 368 nm, ε: 38672 M⁻¹cm⁻¹) of vinamidinium salt 7a of the present invention produced in the above is shown in Fig. 16.

### [Example 17]

### <Production of vinamidinium salt 11a of the present invention>

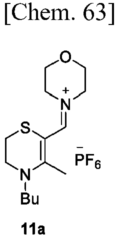

### <Summary of the production method>

As shown in the following reaction formula, first, by the alkylation reaction of thiomorpholine 8, N-alkylthiomorpholine 9 is synthesized, and then by treating N-alkylthiomorpholine 9 with methyllithium, cyclic enamine 10 is prepared. Further, cyclic enamine 10 is reacted with Vilsmeier-type reagent prepared from N-formylamide and phosphorus oxychloride, and by the subsequent conversion to hexafluorophosphate, the vinamidinium salt 11a of the present invention is synthesized.

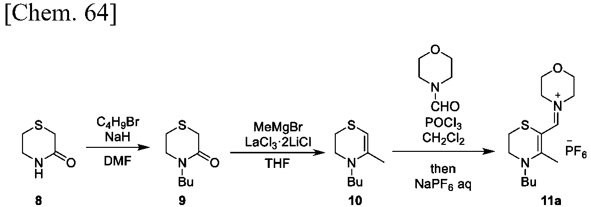

### <Specific production method>

### (Synthesis of compound 9)

Compound 8 (2.39 g, 20.4 mmol) prepared by referring to the literature (H.Ishibashi, M.Uegaki, M,Sakai, Y.Takeda,Tetrahedron,2001,57,2115) was dissolved in distilled and dried N,N-dimethylformamide (21.0 mL) under argon atmosphere. The obtained solution was cooled to 0 °C, added with sodium hydride (in mineral oil 60%, 1.31 g, 32.8 mmol). To this suspension liquid, 1-bromobutane (3.20 mL, 29.7 mmol) was slowly dropped, the reaction mixture was warmed to room temperature, and then stirred for 14 hours. Water was added to the reaction liquid to quench the reaction, and the mixture was extracted 3 times with ethyl acetate. The combined organic layers were washed with brine, then dried over anhydrous sodium sulfate, filtered to concentrate the filtrate. The obtained crude product was purified by silica gel column chromatography (AcOEt/hexane = 1:5) and reduced-pressure distillation (0.7 torr, 134 °C), to obtain 2.75 g (78%) of compound 9 as a colorless transparent oil.

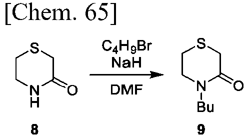

¹H NMR (400 MHz, CDCl₃) δ 3.60 (t, *J* = 5.7 Hz, 2H), 3.41 (t, *J* = 7.5 Hz, 2H), 3. 30 (s, 2H), 2.86 (t, *J* = 5.7 Hz, 2H), 1.59-1.50 (m, 2H), 1.33-1.29 (m, 2H), 0.94 (t, *J* = 7.3Hz, 3H); ¹³C NMR (150 MHz, CDCl₃) δ 166.1, 48.9, 47.6, 30.1, 29.6, 26.4, 1 9.9, 13.7; IR(KBr): 1636 cm⁻¹;

### (Synthesis of compound 10)

Under argon atmosphere, compound 9 (1.00 g, 5.78 mmol) was dissolved in dehydrated tetrahydrofuran (8.80 mL), and added with LaCl₃·2LiCl (0.6 M tetrahydrofuran solution, 12.5 mL, 7.50 mmol) at room temperature. The solution was cooled to -18 °C, and methylmagnesium bromide (0.78 M tetrahydrofuran solution, 9.50 mL, 7.41 mmol) was slowly dropped. The reaction mixture was stirred at -18 °C for 2 hours, and then ice and saturated NH₄Cl aqueous solution were added to the reaction liquid to quench the reaction. After raising the temperature to room temperature, the mixture was extracted 3 times with diethyl ether. The combined organic layers were washed with brine, then dried over anhydrous sodium sulfate, filtered to concentrate the filtrate. The obtained crude product was purified by reduced-pressure distillation (2.0 torr, 150 °C), to obtain 743 mg (76%) of compound 10 as colorless and transparent oil.

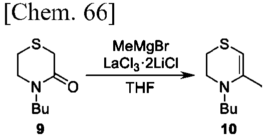

¹H NMR (400 MHz, CDCl₃) δ 4.46 (s, 1H), 3.46-3.43 (m, 2H), 2.93 (t, *J* = 7.5 Hz, 2H), 2.84-2.82 (m, 2H), 1.86 (s, 3H), 1.48-1.40 (m, 2H), 1.33-1.26 (m, 2H), 0.92 (t, *J* = 7.3 Hz, 3H); ¹³C NMR (101MHz, CDCl₃) δ 137.1, 83.7, 51.4, 49.5, 30.6, 24.0, 2 1.8, 20.1, 14.0; IR(KBr): 2957, 1601 cm⁻¹.

### (Synthesis of compound 11a)

Under argon atmosphere, N-formylmorpholine (480 µL, 4.76 mmol) was added to a flask, cooled to 13 °C, and phosphorus oxychloride (90.0 µL, 0.965 mmol) was slowly dropped. The obtained solution was slowly raised the temperature to room temperature, and then dichloromethane (240 µL) solution of compound 10 (109 mg, 0.645 mmol) was dropped. The reaction mixture was raised the temperature to 50 °C, stirred for 3 hours, then cooled to room temperature, and aqueous sodium hexafluorophosphate solution (3.0 M, 860 µL, 2.58 mmol) was added. The reaction mixture was stirred for 15 min., added with water, and extracted 3 times with dichloromethane. The combined organic layers were washed with brine, then dried over anhydrous magnesium sulfate, filtered, and the filtrate was concentrated with an evaporator. Further, the remained N-formylmorpholine was distilled away by using Kugelrohr distillation apparatus, and then the obtained crude product was purified by silica gel chromatography (CHCl₃toCHCl₃:MeOH = 30:1) and washing with EtOH to give 155 mg (58%) of compound 11a as red-colored oil.

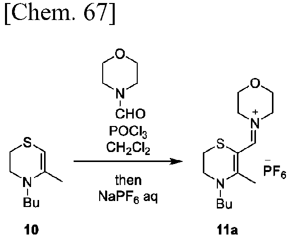

¹H NMR (400 MHz, CDCl₃) δ 7.85 (s, 1H), 3.96-3.89 (m, 6H), 3.83 (t, *J* = 4.8 Hz, 4H), 3.59 (t, *J* = 8.0 Hz, 2H), 2.95 (t, *J* = 4.9 Hz, 2H), 2.51 (s, 3H), 1.72-1.64 (m, 2H), 1.43-1.34 (m, 2H), 0.97 (t, *J* = 7.3 Hz, 3H); ¹³C NMR (101 MHz, CDCl₃) δ 16 7.5, 155.2, 92.0, 66.8, 56.1, 53.2 (2C), 29.7, 23.8, 19.8, 18.3, 13.6; IR(KBr): 2964, 16 07, 1549 cm⁻¹; MS(EI) m/z (%) 269 ([M-PF₆]⁺, 100); HRMS(EI) calced for C₁₄H₂₅N₂ OS [M-PF₆]⁺: 269.1689, found:269.1687.

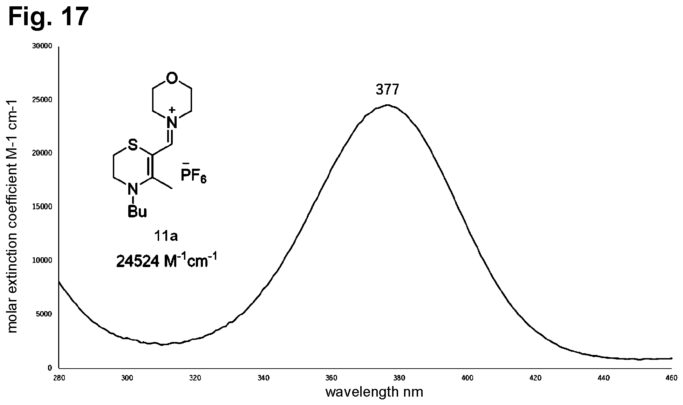

The UV absorbing spectrum (solvent: CHCl₃, λₘₐₓ: 377 nm, ε: 24524 M⁻¹cm⁻¹) of vinamidinium salt 11a of the present invention produced in the above is shown in Fig. 17.

### INDUSTRIAL APPLICABILITY

Since the novel vinamidinium salt of the present invention can be used as an UV-A absorbent (ultraviolet ray inhibitor) in the field of cosmetics, industrial products, etc., it is industrially useful.

## Claims

1. A vinamidinium salt shown by the following formula (1): 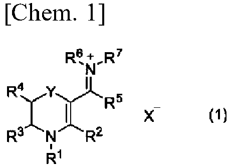
(in formula (1)
R¹, R², R⁵, R⁶ and R⁷ each independently represents a hydrogen atom, an alkyl group with 1 to 10 carbon atoms optionally having a substituent, an alkenyl group with 1 to 10 carbon atoms optionally having a substituent, an alkynyl group with 1 to 10 carbon atoms optionally having a substituent, an aryl group with 6 to 14 carbon atoms optionally having a substituent, an aralkyl group with 7 to 20 carbon atoms optionally having a substituent, or a hydroxy group;
R³ and R⁴ each independently represents a hydrogen atom, an alkyl group with 1 to 10 carbon atoms optionally having a substituent, an alkenyl group with 1 to 10 carbon atoms optionally having a substituent, an alkynyl group with 1 to 10 carbon atoms optionally having a substituent, an alkoxy group with 1 to 10 carbon atoms optionally having a substituent, a hydroxy group or a halogen atom;
Y is O, S, NR⁸, CR⁸R⁹;
R⁸ and R⁹ each independently represents a hydrogen atom, an alkyl group with 1 to 10 carbon atoms optionally having a substituent, an alkenyl group with 1 to 10 carbon atoms optionally having a substituent, an alkynyl group with 1 to 10 carbon atoms optionally having a substituent, an alkoxy group with 1 to 10 carbon atoms optionally having a substituent, an aryl group with 6 to 14 carbon atoms optionally having a substituent, an aralkyl group with 7 to 20 carbon atoms optionally having a substituent, or a hydroxy group;
R¹ to R⁹ can form a cyclic structure by binding to each other; and
X⁻ represents an anion).

2. The vinamidinium salt according to claim 1, wherein R⁶ and R⁷ constitute a nitrogen-containing cyclic group together with a nitrogen atom to which they are bonded.

3. The vinamidinium salt according to claim 2, wherein the nitrogen-containing cyclic group is at least one group selected from an azetidine group, a pyrrolidine group, a piperidine group, an azepane group, a morpholine group, a thiomorpholine group, a thiomorpholine dioxide group, and a piperazine group.

4. The vinamidinium salt according to claim 1, wherein the vinamidinium salt is shown by the following formula (1A):

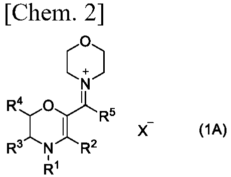

5. The vinamidinium salt according to claim 4, wherein in formula (1A), R¹ represents an alkyl group with 1 to 4 carbon atoms, R² represents a methyl group, R³ to R⁵ represent a hydrogen atom, and X⁻ represents PF₆⁻.

6. A vinamidinium salt shown by the following formula (2). 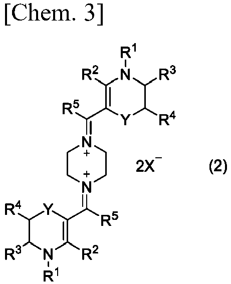
(in formula (2),
R¹, R² and R⁵ each independently represents a hydrogen atom, an alkyl group with 1 to 10 carbon atoms optionally having a substituent, an alkenyl group with 1 to 10 carbon atoms optionally having a substituent, an alkynyl group with 1 to 10 carbon atoms optionally having a substituent, an aryl group with 6 to 14 carbon atoms optionally having a substituent, an aralkyl group with 7 to 20 carbon atoms optionally having a substituent, or a hydroxy group;
R³ and R⁴ each independently represents a hydrogen atom, an alkyl group with 1 to 10 carbon atoms optionally having a substituent, an alkenyl group with 1 to 10 carbon atoms optionally having a substituent, an alkynyl group with 1 to 10 carbon atoms optionally having a substituent, an alkoxy group with 1 to 10 carbon atoms optionally having a substituent, a hydroxy group or a halogen atom;
Y is O, S, NR⁸, CR⁸R⁹;
R⁸ and R⁹ each independently represents a hydrogen atom, an alkyl group with 1 to 10 carbon atoms optionally having a substituent, an alkenyl group with 1 to 10 carbon atoms optionally having a substituent, an alkynyl group with 1 to 10 carbon atoms optionally having a substituent, an alkoxy group with 1 to 10 carbon atoms optionally having a substituent, an aryl group with 6 to 14 carbon atoms optionally having a substituent, an aralkyl group with 7 to 20 carbon atoms optionally having a substituent, or a hydroxy group;
R¹ to R⁵, R⁸ and R⁹ can form a cyclic structure by binding to each other; and
X⁻ represents an anion).

7. The vinamidinium salt according to claim 1 or 6, wherein X⁻ is an anion selected from the group consisting of PF₆⁻, BF₄⁻, halide ion, R^{A}CO₂ (R^{A} represents an organic group with 1 to 20 carbon atoms), and R^{B}SO₄⁻ (R^{B} represents an organic group with 1 to 20 carbon atoms).

8. An UV-A absorbent comprising the vinamidinium salt according to claim 1 or 6.

9. The UV-A absorbent according to claim 8, wherein the UV-A absorbent has transparency.

10. An external preparation for skin comprising the vinamidinium salt according to claim 1 or 6.

11. A glass material comprising the vinamidinium salt according to claim 1 or 6.

12. A resin material comprising the vinamidinium salt according to claim 1 or 6.
